# FASCICULE DE BREVET EUROPEEN

(11) **EP 2 671 607 B1**
(45) Date de publication et mention de la délivrance du brevet: **13.06.2018**
(21) Numéro de dépôt: 13163933.8
(22) Date de dépôt: 16.04.2013
(51) Int. Cl.: A61M 16/00, F16M 13/02, A62B 9/04, F16M 11/04, A61G 12/00, A61G 3/00, A61G 13/10

(54) **Ensemble formé d'un respirateur d'urgence et de son embase murale**
Einheit aus einem Notbeatmungsgerät und seiner Wandhalterung
Assembly of an emergency respirator and the wall-mounted base thereof

(30) Priorité: 07.06.2012 FR 1255314
(43) Date de publication de la demande: 11.12.2013
(73) Titulaire: Air Liquide Medical Systems, 92160 Antony (FR)
(72) Inventeur: Chaffard, Denis, 01170 Gex (FR); Davoine, Romain, 75014 Paris (FR)
(74) Mandataire: Pittis, Olivier

(56) Documents cités:
- DE-A1-102008 039 651
- FR-A- 1 200 711
- FR-A1- 2 906 149
- GB-A- 879 878
- US-A- 5 433 222
- US-A1- 2006 104 014
- US-A1- 2010 126 506
- US-A1- 2010 219 220

## Description

L'invention concerne un ensemble médical formé d'un respirateur d'urgence et de son dispositif-support, c'est-à-dire d'une embase destinée à être fixée à une paroi, tel un mur d'un bâtiment hospitalier ou une paroi de véhicule d'urgence de type ambulance, SAMU..., comprenant un système de connexion électrique permettant une recharge des batteries du respirateur lorsque celui-ci est positionné et verrouillé sur l'embase.

Les dispositifs ou appareils de respiration artificielle, appelés dans le cadre de la présente invention "respirateurs", sont largement utilisés dans les bâtiments hospitaliers ou les unités d'urgence mobiles, tel que les SAMU, les ambulances... pour assister les patients souffrant de défaillances respiratoires.

Classiquement, ces respirateurs sont rangés sur un dispositif-support mural, c'est-à-dire une embase fixée à une paroi, lorsqu'ils ne sont pas utilisés, et en sont retirés par le personnel soignant, lorsqu'ils doivent être utilisés.

Les respirateurs sont dotés de batteries électriques permettant d'assurer leur autonomie électrique. Ces batteries doivent être rechargées plus ou moins fréquemment en fonction de l'intensité de l'utilisation du respirateur.

Actuellement, il existe des supports de fixation murale pour respirateurs médicaux de deux types, à savoir ceux sans système de recharge des batteries du respirateur et résistant à une accélération de 10g, qui est la force d'accélération à laquelle le respirateur/embase doit résister sans se rompre afin d'éviter qu'ils deviennent des projectiles et ne mettent en péril la sécurité des personnes présentes dans les unités d'urgence mobiles, tel que les ambulances ; et par ailleurs, ceux avec système de recharge des batteries du respirateur mais résistant uniquement à une charge de 3g.

Ainsi, on peut citer les documents suivants qui proposent de tels respirateurs : FR-A-2906149, DE-A-102010012641, DE-A-102008064480 et DE-A-102008039651.

Toutefois, ces ensembles formés d'un dispositif de fixation murale et d'un respirateur associé présentent souvent une architecture complexe, ne permettent pas toujours une fixation aisée et rapide du respirateur sur le support et/ou leur raccordement électrique rapide ou aisé.

Par ailleurs, le document FR-A-2906149 propose un ensemble mobile de traitement de patient formé d'un système d'assistance respiratoire et d'une embase-support pouvant être raccordé à une interface côté patient et alimenté en gaz par une bouteille d'oxygène et en courant par un accumulateur d'énergie. Ce dispositif permet de déterminer rapidement la concentration en CO₂ du sang du patient et alimenter le patient en O₂ quand cette concentration en CO₂ est trop élevée. Le dispositif peut comprendre un système de fixation permettant de le fixer à une paroi, notamment dans un véhicule d'urgence. La fixation se fait par mise en place, basculement et encliquetage d'un axe de verrouillage dans une plaque- support. Ce dispositif supporte des charges de 3g, voire de 6g ou plus.

Par ailleurs, le document US-A-5,433,22 enseigne un dispositif servant à attacher un patient qui doit être immobilisé. Ce dispositif, qui est destiné à venir se fixer à des rails d'une table de soins ou d'examens, comprend un bras pivotant sur la partie arrière duquel vient appuyer un ressort de rappel. Sa partie avant comprend une griffe venant accrocher un rail. Une sangle de maintien du patient est fixée audit dispositif.

De là, le problème qui se pose est de pouvoir disposer d'un ensemble amélioré formé d'un dispositif de fixation murale ou embase et d'un respirateur associé comprenant un système de recharge des batteries et pouvant avantageusement résister à une accélération de 10g selon les 3 directions (x, y, z), permettant une fixation solidaire, rapide et aisée pour l'utilisateur du respirateur au dispositif-support, que ledit dispositif-support soit fixé sur un mur ou une paroi de bâtiment hospitalier, ou une paroi d'un véhicule d'urgence ou analogue, telle une ambulance, un véhicule de pompier ou du SAMU..., directement ou par l'intermédiaire d'un rail.

La présente invention vise donc à proposer un ensemble formé d'un dispositif-support ou embase et d'un respirateur associé permettant d'obtenir non seulement une fixation facile et rapide du respirateur sur son support mais aussi un raccordement électrique simultané du respirateur à une source de courant alimentant l'embase.

La solution est alors un ensemble respirateur/embase comprenant un respirateur, c'est-à-dire un dispositif ou appareil de respiration artificielle, et une embase, c'est-à-dire un dispositif-support mural, apte à et conçue pour recevoir et porter le respirateur, ladite embase comprenant un système de fixation permettant sa fixation, directe ou indirecte, à une paroi, caractérisé en ce qu'il comporte en outre :
- un système d'accrochage permettant une mise en position de couplage du respirateur sur l'embase,
- un système de verrouillage permettant de bloquer le respirateur en une position de verrouillage sur l'embase, subséquemment à la mise en position de couplage, la position de verrouillage étant distincte de la position de couplage, le système de verrouillage comprenant des premiers moyens de verrouillage portés par le respirateur, et des seconds moyens de verrouillage portés par l'embase et aptes à et conçus pour coopérer avec les premiers moyens de verrouillage de manière à permettre une fixation solidaire en position de verrouillage du respirateur à l'embase, et
- un système de connexion électrique permettant d'assurer une connexion électrique entre le respirateur et l'embase, subséquemment à la mise en position de couplage et simultanément ou quasi-simultanément à la mise en position de verrouillage, la position de verrouillage étant distincte de la position de couplage, et
- un système de connexion électrique permettant d'assurer une connexion électrique entre le respirateur et l'embase, subséquemment à la mise en position de couplage et simultanément ou quasi-simultanément à la mise en position de verrouillage,
caractérisé en ce que les premiers moyens de verrouillage portés par le respirateur comprennent une butée, et les seconds moyens de verrouillage portés par l'embase comprennent un cliquet pivotant, ledit cliquet coopérant avec ladite butée pour maintenir le respirateur solidaire de l'embase en position de verrouillage, le cliquet étant porté par un support de cliquet et actionné par un ressort qui appuie normalement sur une partie avant du cliquet afin d'assurer ledit maintien solidaire du respirateur sur l'embase en ladite position de verrouillage. Selon le cas, l'ensemble respirateur/embase selon la présente invention peut comprendre l'une ou plusieurs des caractéristiques techniques suivantes :
- le système d'accrochage comprenant des premiers moyens d'accrochage portés par le respirateur et des seconds moyens d'accrochage portés par l'embase et aptes à et conçus pour coopérer avec lesdits premiers moyens d'accrochage de manière à permettre une mise en position et un accrochage du respirateur à l'embase.
- le système de connexion électrique comprend des premiers moyens contacteurs-électriques portés par le respirateur, et des seconds moyens contacteurs-électriques portés par l'embase et aptes à et conçus pour coopérer avec les premiers moyens contacteurs-électriques de manière à réaliser une connexion électrique du respirateur à l'embase, lorsque le respirateur est en position de verrouillage.
- il comporte en outre des moyens de libération coopérant avec les seconds moyens de verrouillage de manière à permettre un désengagement et une libération desdits premiers moyens de verrouillage, et à permettre en outre simultanément ou quasi-simultanément une déconnexion desdits premiers et seconds moyens contacteurs-électriques, les uns des autres.
- lorsque le respirateur est en position de couplage sur l'embase, ledit respirateur est mobile en pivotement ou basculement par rapport à l'embase.
- les premiers moyens d'accrochage du respirateur comprennent un premier logement et les seconds moyens d'accrochage de l'embase comprennent un élément faisant saillie apte à et conçu pour venir s'insérer, au moins en partie, dans ledit logement.
- le premier logement est aménagé dans une platine-support portée par une face arrière du respirateur, de préférence le premier logement est un évidement ou une ouverture pratiquée dans la platine-support.
- la butée est portée par la platine-support fixée à la face arrière du respirateur.
- les premiers moyens contacteurs-électriques portés par le respirateur comprennent un ou plusieurs contacteurs, de préférence des contacteurs femelles, et les seconds moyens contacteurs-électriques, portés par l'embase comprennent un ou plusieurs contacteurs, de préférence des contacteurs mâles.

- la position de verrouillage est angulairement décalée de la position de couplage.
- les moyens de libération comprennent un levier actionnable par l'utilisateur.
- lorsque l'utilisateur actionne le levier, ledit levier agit sur le cliquet de manière à le désengager de la butée.
- l'embase est raccordée à une alimentation électrique, de préférence une alimentation électrique convertissant le courant du secteur, i.e. un courant alternatif de 110 V ou de 220-230 V, en un courant continu de 12-24 V.
- le respirateur comprend un ou plusieurs moyens de stockage électrique rechargeables, en particulier une ou plusieurs batteries électriques rechargeables.
- le ressort est muni d'une tête de ressort.
- le cliquet pivote autour de son axe de cliquet BB porté par le support de cliquet tout en y étant maintenu.
- le ressort permet une résistance à une force de traction de 10g appliquée au respirateur.
- le ressort de cliquet appuie normalement sur la partie avant du cliquet ou tête de cliquet, par l'intermédiaire de la tête de ressort afin de le maintenir dans la position dite « verrouillée » ou de verrouillage.
- la forme de la partie avant ou tête du cliquet est arrondie afin de permettre une connexion mécanique aisée et rapide avec le respirateur, par simple mise en contact puis appui manuel sur le respirateur muni de la platine de fixation.

L'invention concerne par ailleurs un respirateur d'un ensemble selon l'invention comprenant une platine-support fixée à la face arrière du respirateur, ainsi qu'une embase d'un ensemble selon l'invention.

En d'autres termes, l'ensemble respirateur/embase murale selon la présente invention repose sur une combinaison d'éléments et de moyens comprenant un système d'accrochage permettant de réaliser un couplage mécanique rapide et aisé du respirateur à son support mural, c'est-à-dire l'embase, un système de connexion mécanique servant à verrouiller, c'est-à-dire à maintenir le respirateur bloqué sur l'embase jusqu'à une accélération maximum de 10g, et un système de connexion électrique rapide permettant de raccorder électriquement le respirateur à l'embase qui est lui-même alimenté en courant électrique par le réseau, de manière soit à assurer le fonctionnement de l'appareil (sans batterie), soit à pouvoir recharger les batteries du respirateur dès que celui-ci est verrouillé sur l'embase.

L'invention va maintenant être mieux comprise grâce à la description suivante faite en références aux Figures annexées parmi lesquelles :
- la FIG. 1 illustre un mode de réalisation, vu de face, d'une embase murale d'un ensemble respirateur/embase selon la présente invention,
- les FIG. 2 et 4 sont des vues de côté (droit et gauche) de l'embase de la FIG.1,
- la FIG. 3 est une vue de la face arrière d'un respirateur apte à venir se raccorder à l'embase selon la FIG.1,
- la FIG. 5 illustre le respirateur, en position de couplage, d'un ensemble respirateur/embase selon la présente invention,
- la FIG. 6 illustre le respirateur, en position de verrouillage, d'un ensemble embase/respirateur selon la présente invention,
- la FIG. 7 schématise une étiquette apposée sur l'ensemble de l'invention illustrant la mise en place du respirateur de la FIG. 3 sur l'embase de la FIG.1,
- la FIG. 8 schématise un mode de réalisation des moyens de verrouillage de l'embase selon l'invention,
- la FIG. 9 est analogue à la FIG. 8 mais schématise en plus la coopération avec le respirateur de l'ensemble selon l'invention,
- la FIG. 10 schématise un mode de réalisation des moyens de connexion électrique d'un ensemble selon l'invention, et

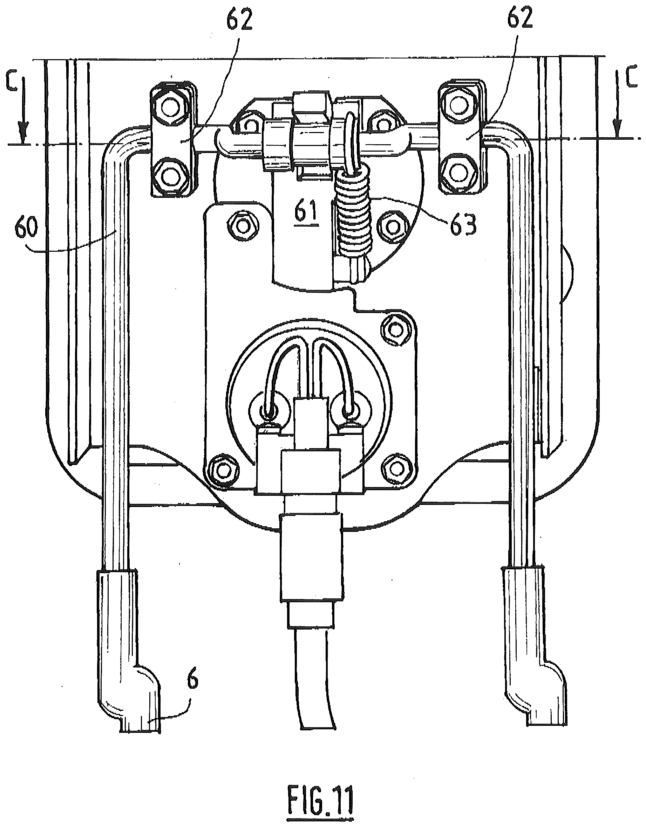

- la FIG. 11 illustre un mode de réalisation des moyens de libération permettant le déverrouillage du respirateur et sa désolidarisation de l'embase selon l'invention.

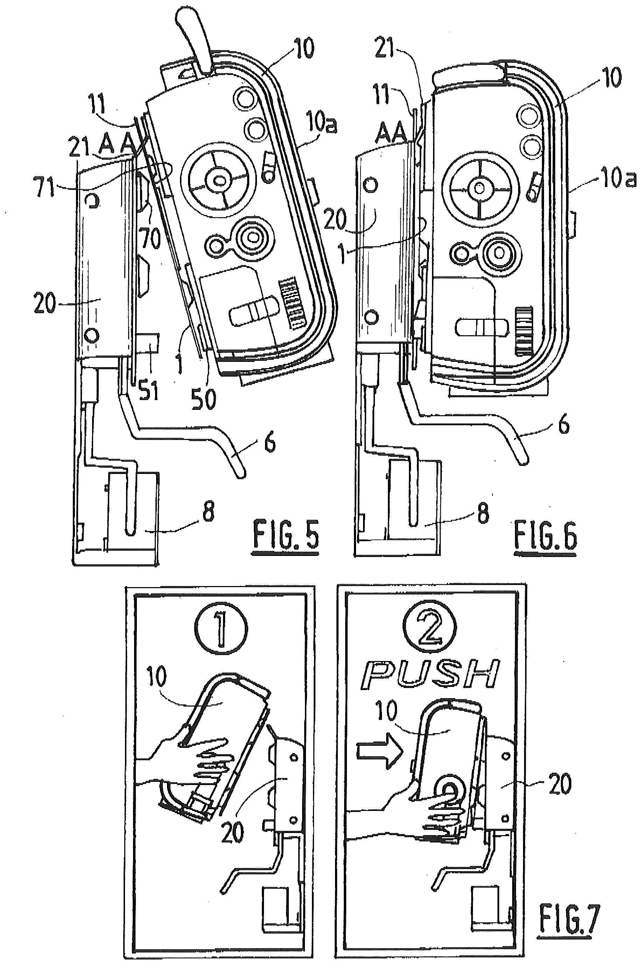

Les Figures 5 et 6 schématisent un mode de réalisation d'un ensemble respirateur/embase selon la présente invention comprenant un respirateur 10, c'est-à-dire un dispositif ou appareil de respiration artificielle, et une embase murale 20 faisant office de dispositif-support en étant apte à et conçue pour recevoir et porter le respirateur 10.

Le respirateur 10 comprend une face avant 10a avec, de manière classique, un ou des organes de réglages, tel que boutons, touches, curseurs..., un ou des afficheurs, tel des écrans de visualisation, voyants, ...

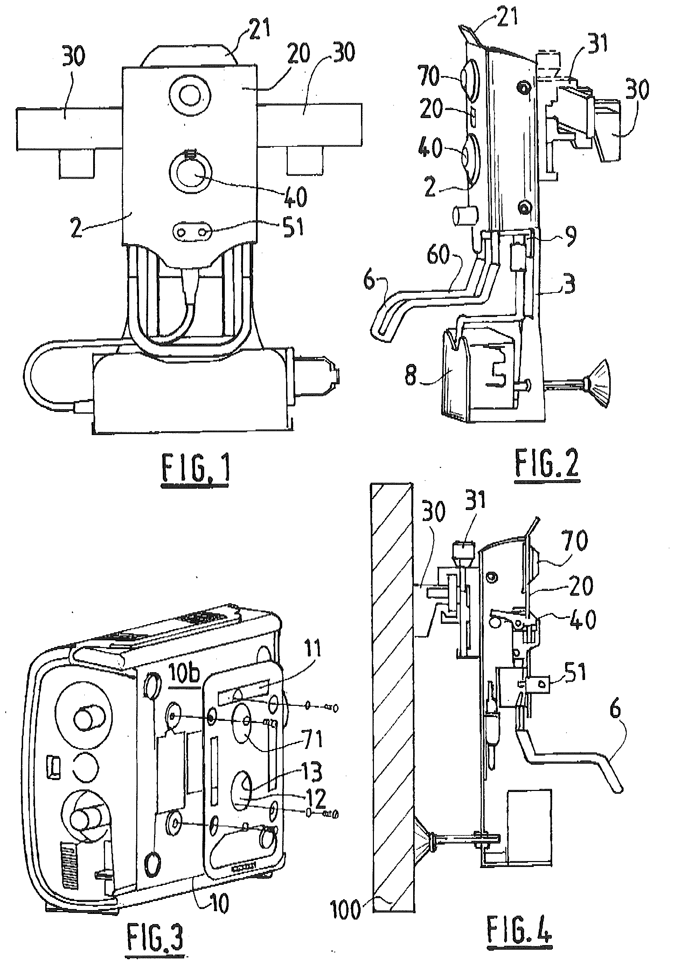

Par ailleurs, l'embase 20 faisant office de dispositif-support mural, détaillée sur les Figures 1, 2 et 4, comprend un système de fixation 31 permettant sa fixation à une paroi, par exemple un mur 100 de bâtiment hospitalier comme schématisé en Figure 4.

Ici, l'embase 20 est fixée à une paroi 100, via un rail porteur 30 sur lequel vient se raccorder le système de fixation 31, par exemple le système de fixation 31 peut comprendre deux mords et une vis de serrage. Tout autre système de fixation 31 adapté convient également. Le système de fixation 31 est porté par la face ou châssis arrière 3 du support 20, comme illustré en Figures 2 et 4.

L'ensemble respirateur/embase de l'invention comprend en outre un système d'accrochage 1, 11, 2, 21 permettant une mise en position de couplage, aisée et rapide, du respirateur 10 sur l'embase 20 par simple accrochage du respirateur 10 à l'embase 20, comme illustré sur les Figures 5 à 7.

Plus précisément, le système d'accrochage 1, 11, 2, 21 comprend des premiers moyens d'accrochage 1, 11 portés par le respirateur 10 et des seconds moyens d'accrochage 2, 21 portés par le dispositif-support ou embase 20, et aptes à et conçus pour coopérer avec lesdits premiers moyens d'accrochage 1, 11 de manière à permettre une mise en position aisé et rapide et un accrochage du respirateur 10 à l'embase 20.

Les premiers moyens d'accrochage 1, 11 portés par le respirateur 10 comprennent par exemple un premier logement ou évidement 11, en particulier une ouverture ou découpe, aménagé dans une plaque métallique ou platine de fixation arrière 1 fixée, par exemple par vissage à l'aide ici de quatre vis disposées suivant une géométrie de type VESA, à la face arrière 10b du respirateur comme détaillé en Figure 3.

La platine de fixation arrière 1 sert donc d'interface entre le respirateur 10 et la station murale, c'est-à-dire le dispositif-support 20. Elle est fixée à demeure sur le respirateur 10.

Il est à noter que le premier logement ou évidement 11 peut être aussi aménagé directement dans la paroi de la face arrière 10b du respirateur 10 et que dans cet autre mode de réalisation (non montré), on peut se passer de la platine de fixation 1.

Par ailleurs, les seconds moyens d'accrochage 2, 21 portés par le dispositif-support 20 comprennent, quant à eux, au moins une patte d'accrochage 21, c'est-à-dire un élément en saillie, configurée et dimensionnée par permettre son insertion dans le logement 11 du respirateur 1.

Cette patte d'accrochage 21 peut être avantageusement formée par une portion en saillie d'une plaque formant face avant ou châssis avant 2 de l'embase 20, comme illustré en Figure 2. En effet, comme illustré en Figures 5 à 7, la mise en place du respirateur 10 sur l'embase murale 20 par un utilisateur se fait simplement par insertion de la patte d'accrochage 21 en saillie dans le logement ou ouverture 11 de l'embase 20.

Le respirateur 10 se trouve alors en position de couplage, c'est-à-dire qu'il est connecté de manière non solidaire au support 20 en y étant retenu uniquement grâce à la coopération entre la patte d'accrochage 21 et le logement 11. Dans cette position de couplage, le respirateur 10 peut être retiré du support mural 20 simplement en retirant la patte d'accrochage 11 de son logement 21.

En d'autres termes, les premiers moyens d'accrochage 1, 11 comprenant le logement 11 portés par la plaque arrière 1 du respirateur 10 coopèrent avec les seconds moyens d'accrochage 2, 21 comprenant la patte d'accrochage 21 portés par la face avant 2 du dispositif-support ou embase 20 de manière à assurer une mise en position et un accrochage corrects du respirateur 10 sur l'embase 20 murale. Toutefois, dans cette position dit position de couplage, le respirateur 10 n'est pas retenu de manière solidaire de l'embase 20.

Pour faciliter la mise en position de couplage du respirateur 10 sur l'embase 20, on prévoit avantageusement un système de détrompage 70, 71 sur le respirateur 10 et sur l'embase 20. Par exemple, on peut prévoir un élément en saillie 70 porté par la face ou châssis avant 2 de l'embase 2 comme illustré sur les Figures 1, 2 et 4, lequel vient s'engager dans un troisième logement ou évidement 71, de forme au moins partiellement complémentaire, aménagé dans la plaque ou platine arrière 1 du ventilateur 10, comme illustré sur les Figures 3 et 5 notamment.

Une fois le ventilateur 10 couplé à l'embase 20 par insertion de la patte d'accrochage 21 dans le premier logement 11 de la platine 1, l'élément en saillie 70 porté par le châssis avant 2 de l'embase 20 permet d'assurer un positionnement correct et un bon alignement du respirateur 10 sur l'embase 20, en venant pénétrer dans le troisième logement 71 et en venant s'y centrer, lorsque l'utilisateur opère un basculement angulaire du ventilateur 10 de la position de couplage à la position de verrouillage, en venant exercer une force d'appui (flèche sur Fig. 7) sur la partie inférieure du respirateur 10, comme schématisé en Figure 7.

Ensuite, afin de bloquer ensuite le respirateur 10, en une position de verrouillage, sur l'embase 20, c'est-à-dire de les rendre solidaires l'un de l'autre, il est prévu par ailleurs un système de verrouillage 1, 12, 13, 40-45. Ce système de verrouillage 1, 12, 13, 40-45 permet d'assurer une bonne connexion mécanique de l'ensemble respirateur/embase murale, c'est-à-dire un maintien en position fixe et solidaire du respirateur 10 sur l'embase 20, après mise en position de couplage.

La position de verrouillage est donc une position du respirateur 10 sur le support 20, qui est distincte de la position de couplage puisqu'en position de verrouillage, le respirateur 10 est maintenu fermement sur l'embase murale 20 et ne peut en être retiré, sans actionnement préalable de moyens de libération, comme détaillé ci-après.

Schématiquement, la position de verrouillage est une position angulairement décalée par rapport à la position de couplage et l'on passe donc de ladite position de couplage à celle de verrouillage en opérant un léger pivotement ou basculement du respirateur 10 autour de l'axe AA (cf. Figures 5 et 6) qui se forme lors de l'insertion de la patte d'accrochage 21 au sein du logement 11.

Plus précisément, le système de verrouillage 1, 12, 13, 40-45 assurant un maintien en position fixe et solidaire du respirateur 10 sur l'embase 20 comprend un ensemble de pièces ou d'éléments mécaniques permettant une connexion mécanique du respirateur 10 muni de la platine de fixation 1 sur le support mural 20.

Cet ensemble de pièces ou d'éléments mécaniques qui forme le système de verrouillage 1, 12, 13, 40-45 comprend des premiers moyens de verrouillage 1, 12, 13 portés par le respirateur 10 coopérant avec des seconds moyens verrouillage 40-45 portés par l'embase 20 de manière à garantir une fixation et un maintien solidaire en position de verrouillage, du respirateur 10 sur le dispositif-support ou embase 20.

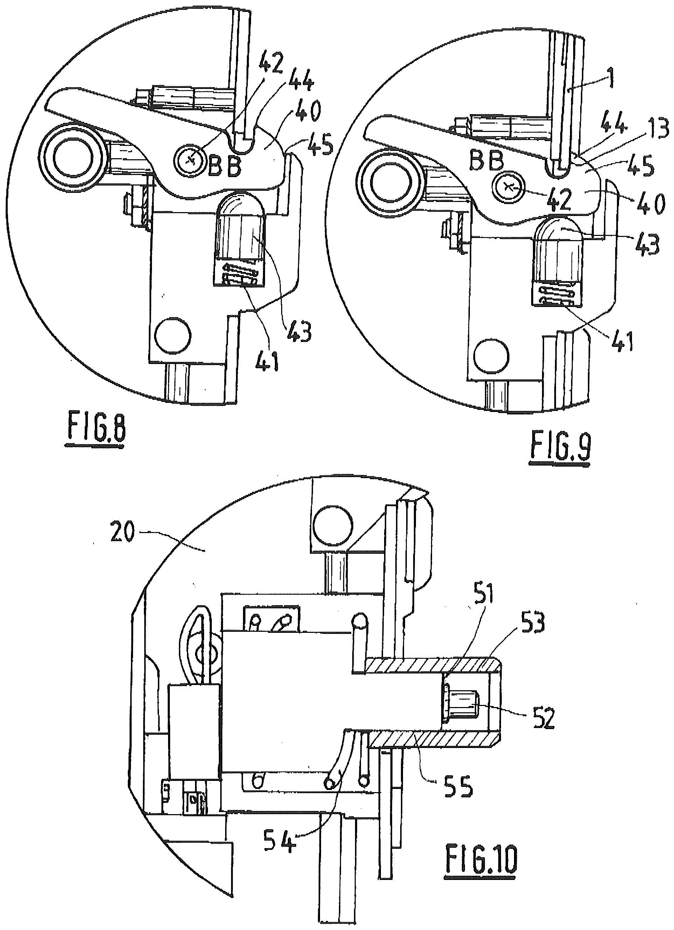

Les seconds moyens de verrouillage 40-45 portés par l'embase 20 comprennent avantageusement un cliquet pivotant 40 porté par un support de cliquet 42 et actionné par un ressort 41 muni d'une tête de ressort 43, comme détaillé en Figure 8. Le cliquet 40 pivote autour de son axe de cliquet BB porté par le support de cliquet 42, tout en y étant maintenu. Un bon dimensionnement du ressort 41 permet une résistance à une force de traction de 10g appliquée au respirateur.

Le ressort 41 de cliquet appuie normalement sur la partie avant 45 du cliquet 40, c'est-à-dire la tête du cliquet 40, par l'intermédiaire de la tête de ressort 43 afin de le maintenir dans la position dite « verrouillée » ou de verrouillage. La forme de la partie avant ou tête 45 du cliquet 40 est préférentiellement arrondie afin de permettre une connexion mécanique aisée et rapide avec le respirateur 10 et ce, par simple mise en contact puis appui manuel sur le respirateur 10 muni de la platine de fixation 1.

En effet, la platine de fixation 1 située sur la face arrière 10b du respirateur 10 comprend des premiers moyens de verrouillage coopérant avec les seconds moyens de verrouillage de l'embase 20, à savoir avec notamment le cliquet 40 et le ressort de cliquet 41.

Plus précisément, les premiers moyens de verrouillage du respirateur 10 comprennent une butée 13 venant coopérer avec la griffe d'accrochage 44 portée par la tête ou partie avant 45 du cliquet 40 pour assurer un maintien solidaire du respirateur 10 sur l'embase murale 20 en position de verrouillage, comme illustré en Figures 8 et 9.

Une butée 13 peut être, comme montré en Figure 3, formée par une partie de la paroi de la platine arrière 1 du respirateur 10. En particulier, la platine arrière 1 comprend ici un deuxième logement ou évidement 12 dimensionné et configuré pour recevoir notamment le cliquet 40, comme illustré en figure 9, une partie de la paroi de ladite platine arrière 1 située en périphérie immédiatement de l'évidement 12 constituant ladite butée 13 sur laquelle vient appuyer la griffe d'accrochage 44 du cliquet 40, lorsque le respirateur 10 est en position de verrouillage (Fig. 6 et 9).

Par ailleurs, l'ensemble respirateur/embase de l'invention comprend aussi un système de connexion électrique 50, 51 permettant d'assurer une connexion électrique entre le respirateur 10 et l'embase 20, subséquemment à la mise en position de couplage et simultanément ou quasi-simultanément à la mise en position de verrouillage, par basculement ou pivotement du respirateur 10 en direction de l'embase murale 20, comme illustré en Figures 5 à 7 notamment.

Le système de connexion électrique 50, 51 comprend des premiers moyens contacteurs-électriques 50 portés par le respirateur 10, coopérant avec des seconds moyens contacteurs-électriques 51 portés par l'embase 20 de manière à réaliser une connexion électrique du respirateur 10 audit l'embase 20, lorsque le respirateur 10 est en position de verrouillage.

En d'autres termes, lorsque le respirateur 10 est basculé par un utilisateur de la position de couplage à la position de verrouillage, les premiers et seconds moyens contacteurs-électriques 50, 51 viennent se raccorder les uns aux autres pour obtenir une continuité électrique entre le respirateur 10 et l'embase 20 alimentée en courant électrique par le secteur, et assurer ainsi le chargement ou rechargement des batteries électriques du respirateur 10.

Le système de connexion électrique 50, 51 comprend préférentiellement au moins deux contacts électriques et un ou plusieurs câbles électriques permettant l'interfaçage électrique entre l'alimentation et le respirateur 10.

Comme détaillé en Figure 10, le système de connexion électrique 50, 51 comprend un support de prise, deux contacts électriques 52 de type mâle sur ressort, compensant les éventuels écarts de positionnement, reliés par un toron 55 à l'embase 20 qui est elle-même reliée à une alimentation en courant, convertissant par exemple le courant du secteur, i.e. 110 V ou 220-230 V alternatif en 12-24 V continu. Il comporte en outre un ou des cache-contact 53, et un ou plusieurs ressorts d'expulsion 54.

Le (les) cache-contact 53 permet la protection de contacts électriques 52 vis-à-vis de l'environnement et protège ainsi l'utilisateur d'éventuels dangers électriques. Il permet aussi, avec le (ou les) ressort d'expulsion 54, le dégagement du respirateur 10, lors de sa déconnexion mécanique de l'embase 20.

Du côté du respirateur 10, les contacts électriques 50 sont de type femelle et reçoivent les deux contacts électriques 52 mâles de l'embase 10 qui viennent s'y connecter par insertion mécanique.

L'alimentation électrique 8 reliée au secteur est connectée (en 9) à l'embase 20 murale et aux contacts électriques des seconds moyens contacteurs-électriques 51 portés par l'embase 20. La bonne connexion électrique est signalée par un voyant lumineux placé en face avant de l'alimentation électrique 8.

L'alimentation électrique 8 peut être enlevée du dispositif-support ou embase par un système d'attache rapide. Par ailleurs, l'alimentation électrique 8 peut être intégrée à demeure dans le châssis arrière 3.

Le respirateur 10 comporte quant à lui une ou des batteries électriques assurant la fourniture de la puissance nécessaire à son fonctionnement lorsqu'il est dissocié de l'embase 20, par exemple lui assurant une autonomie de fonctionnement d'au moins 1 à 2 heures, voire plus.

De plus, le respirateur 10 comporte sur sa face avant 10a, un voyant lumineux indiquant le bon chargement ou rechargement des batteries. Cette information peut être déportée sur le dispositif-support ou embase 20 par un voyant lumineux.

L'ensemble de l'invention comporte en outre des moyens de libération 60, 6 coopérant avec les seconds moyens de verrouillage 40-45 de manière à permettre un désengagement et une libération desdits premiers moyens de verrouillage 1, 12, 13, et à permettre en outre simultanément ou quasi-simultanément une déconnexion desdits premiers et seconds moyens contacteurs-électriques 50, 51, les uns des autres.

Avantageusement, les moyens de libération 60, 6 comprennent un levier ou poignée 6 actionnable par l'opérateur qui se déplace, après actionnement, entre au moins une première position dite « de verrouillage », dans laquelle elle n'agit pas sur les seconds moyens de verrouillage 40-45 et dans laquelle le respirateur 10 est en position de verrouillage, et au moins une deuxième positon dite « de libération » dans laquelle elle interagit avec les seconds moyens de verrouillage 40-45 pour libérer le respirateur 10 de manière à pouvoir le retirer de l'embase 20. Le cache contact 53 revient alors à sa position initiale afin d'assurer sa fonction de sécurité.

En fait, l'actionnement à une main par l'utilisateur de la poignée ou levier 6, engendre un déverrouillage des systèmes de connexions mécanique et électrique, et permet à l'utilisateur de retirer le respirateur 10 de son support mural 20.

Pour le déverrouillage, la poignée 6 actionne par l'intermédiaire d'un manchon soudé 61, le cliquet 40. Celle-ci pivote sur l'axe CC dans des pontets 62. Elle est munie d'un ressort de rappel 63, dimensionné pour garantir la position verrouillée de la poignée 6 lors d'une accélération de 10g, fixé sur le support de cliquet 42 par une goupille

En définitive, un ensemble ventilateur/embase selon la présente invention est particulièrement avantageux car il permet une mise en place rapide et aisée du ventilateur 10 sur son embase 20 en deux étapes majeures successives, comme illustré en Figure 7, à savoir :
- d'abord, une mise en position mécanique initiale du ventilateur sur son support par simple insertion de la patte d'accrochage 21 dans le premier logement ou ouverture 11 de la platine 1 du ventilateur 10 (Position (1) en Fig. 7),
- puis, une connexion mécanique et électrique simultanée ou quasi-simultanée du système avec verrouillage du ventilateur 10 sur l'embase 20, lorsque l'utilisateur pousse le ventilateur 10 en direction de l'embase (Position (2) en Fig. 7 et Flèche « PUSH ») en engendrant alors un actionnement du cliquet 40 par la platine 1 et sa coopération avec la butée 13, et simultanément un retrait du cache-contacts, et une connexion électrique grâce aux contacts électriques 52 males de l'embase 20 qui rentrent en connexion avec les contacts femelles 51 du ventilateur 10.

Lorsque l'utilisateur souhaite utiliser, hors de son dispositif support, le respirateur, la déconnexion mécanique et électrique du ventilateur 10 de l'embase 20 sont également simultanées et s'opèrent lors de l'actionnement de la poignée ou levier 6 par l'utilisateur, laquelle agit alors sur le cliquet 40 qui est basculé en libérant alors la platine 1, et le cache-contacts pousse le ventilateur 10, qui revient dans la position mécanique initiale, c'est-à-dire la position de couplage non verrouillée.

L'ensemble embase murale/respirateur selon la présente invention peut être utilisée pour ventiler tout type de patient nécessitant une assistance respiratoire, notamment en cas d'intervention en urgence.

## Revendications

1. Ensemble respirateur/embase comprenant un respirateur (10) et une embase (20) apte à et conçue pour recevoir et porter le respirateur (10), ladite embase (20) comprenant un système de fixation (31) permettant sa fixation, directe ou indirecte, à une paroi, ledit ensemble respirateur/embase comportant en outre :
- un système d'accrochage (1, 11 ; 2, 21) permettant une mise en position de couplage du respirateur (10) sur l'embase (20),
- un système de verrouillage (1, 12, 13 ; 40-45) permettant de bloquer le respirateur (10) en une position de verrouillage sur l'embase (20), subséquemment à la mise en position de couplage, la position de verrouillage étant distincte de la position de couplage, le système de verrouillage (1, 12, 13 ; 40-45) comprenant des premiers moyens de verrouillage (1, 12, 13) portés par le respirateur (10), et des seconds moyens de verrouillage (40-45) portés par l'embase (20) et aptes à et conçus pour coopérer avec les premiers moyens de verrouillage (1, 12, 13) de manière à permettre une fixation solidaire en position de verrouillage du respirateur (10) à l'embase (20), et
- un système de connexion électrique (51-55) permettant d'assurer une connexion électrique entre le respirateur (1) et l'embase (20), subséquemment à la mise en position de couplage et simultanément ou quasi-simultanément à la mise en position de verrouillage, **caractérisé en ce que** :
- les premiers moyens de verrouillage (1, 12, 13) portés par le respirateur (10) comprennent une butée (13), et
- les seconds moyens de verrouillage (40-45) portés par l'embase (20) comprennent un cliquet pivotant (40), ledit cliquet (40) coopérant avec ladite butée (13) pour maintenir le respirateur (10) solidaire de l'embase (20) en position de verrouillage, le cliquet (40) étant porté par un support de cliquet (42) et actionné par un ressort (41) qui appuie normalement sur une partie avant (45) du cliquet (40) afin d'assurer ledit maintien solidaire du respirateur (10) sur l'embase (20) en ladite position de verrouillage.

2. Ensemble selon l'une des revendications précédentes, **caractérisé en ce que** le système d'accrochage (1, 11 ; 2, 21) comprend des premiers moyens d'accrochage (11) portés par le respirateur (10) et des seconds moyens d'accrochage (2, 21) portés par l'embase (20) et aptes à et conçus pour coopérer avec lesdits premiers moyens d'accrochage (1, 11) de manière à permettre une mise en position et un accrochage du respirateur (10) à l'embase (20).

3. Ensemble selon l'une des revendications précédentes, **caractérisé en ce que** le ressort (41) est muni d'une tête de ressort (43) venant appuyer normalement sur la partie avant (45) du cliquet (40) afin de le maintenir en position de verrouillage.

4. Ensemble selon l'une des revendications précédentes, **caractérisé en ce que** la partie avant (45) du cliquet (40) est de forme arrondie.

5. Ensemble selon l'une des revendications précédentes, **caractérisé en ce que** la partie avant (45) du cliquet (40) comprend une griffe d'accrochage (44) apte à venir coopérer avec la butée (13) pour assurer un maintien solidaire du respirateur (10) sur l'embase (20) en position de verrouillage.

6. Ensemble selon l'une des revendications précédentes, **caractérisé en ce que** la butée (13) est portée par la platine-support (1) fixée à la face arrière du respirateur (10).

7. Ensemble selon l'une des revendications précédentes, **caractérisé en ce que** la butée (13) est formée par une partie de la paroi de la platine-support (1) arrière du respirateur (10).

8. Ensemble selon l'une des revendications précédentes, **caractérisé en ce que** la platine arrière (1) comprend un deuxième logement ou évidement (12) dimensionné et configuré pour recevoir le cliquet (40).

9. Ensemble selon la revendication 8, **caractérisé en ce qu'**une partie de la paroi de ladite platine arrière (1) située en périphérie immédiate du deuxième évidement (12) constitue la butée (13) sur laquelle vient appuyer la griffe d'accrochage (44) du cliquet (40), lorsque le respirateur (10) est en position de verrouillage.

10. Ensemble selon l'une des revendications précédentes, **caractérisé en ce qu'**il comprend des moyens de libération (60, 6) comprenant un levier (6) actionnable par l'utilisateur apte à se déplacer, après actionnement, entre :
- au moins une première position de verrouillage, dans laquelle le levier (6) n'agit pas sur les seconds moyens de verrouillage (40-45) et le respirateur (10) est en position de verrouillage, et
- au moins une deuxième positon de libération, dans laquelle le levier (6) interagit avec les seconds moyens de verrouillage (40-45) pour libérer le respirateur (10) de manière à pouvoir le retirer de l'embase (20).

11. Ensemble selon la revendication précédente, **caractérisé en ce que** lorsque le levier (6) est actionné par l'utilisateur, ledit levier (6) agit en engendrant un déverrouillage des systèmes de connexions mécanique et électrique.

12. Ensemble selon l'une des revendications 10 ou 11, **caractérisé en ce que** le levier (6), en pivotant, actionne le cliquet (40) par l'intermédiaire d'un manchon soudé (61).

13. Embase (20) selon la revendication 1 d'un ensemble selon l'une des revendications précédentes, **caractérisée en ce qu'**elle comporte un système de fixation (31) permettant sa fixation, directe ou indirecte, à une paroi, des seconds moyens d'accrochage (2, 21), des seconds moyens de verrouillage (40-45) et des seconds moyens contacteurs-électriques (51).

## Patentansprüche

1. Atemschutzgerät-/Basiseinheit, umfassend ein Atemschutzgerät (10) und eine Basis (20), die ausgelegt und entworfen ist, um das Atemschutzgerät (10) aufzunehmen und zu tragen, wobei die Basis (20) ein Fixierungssystem (31) umfasst, das ihre direkte oder indirekte Fixierung an eine Wand ermöglicht, wobei die Atemschutzgerät-/Basiseinheit weiter umfasst:
- ein Aufhängesystem (1, 11; 2, 21), das eine Kupplungspositionierung des Atemschutzgeräts (10) auf der Basis (20) ermöglicht,
- ein Verriegelungssystem (1, 12, 13; 40 - 45), das ermöglicht, das Atemschutzgerät (10) in einer Verriegelungsposition auf der Basis (20) nach der Kupplungspositionierung zu blockieren, wobei die Verriegelungsposition verschieden von der Kupplungsposition ist, wobei das Verriegelungssystem (1, 12, 13; 40 - 45) erste Verriegelungsmittel (1, 12, 13), getragen vom Atemschutzgerät (10), und zweite Verriegelungsmittel (40 - 45), getragen von der Basis (20), umfasst und ausgelegt und entworfen, um mit den ersten Verriegelungsmitteln (1, 12, 13) zusammenzuarbeiten, um eine fest verbundene Fixierung in der Verriegelungsposition des Atemschutzgeräts (10) an der Basis (20) zu ermöglichen, und
- ein System zur elektrischen Verbindung (51 - 55), das ermöglicht, eine elektrische Verbindung zwischen dem Atemschutzgerät (1) und der Basis (20) nach der Kupplungspositionierung und gleichzeitig oder fast gleichzeitig mit der Verriegelungspositionierung sicherzustellen,
**dadurch gekennzeichnet, dass**:
- die ersten Verriegelungsmittel (1, 12, 13), getragen vom Atemschutzgerät (10), einen Anschlag (13) umfassen, und
- die zweiten Verriegelungsmittel (40 - 45), getragen von der Basis (20), eine schwenkbare Klinke (40) umfassen, wobei die Klinke (40) mit dem Anschlag (13) zusammenarbeitet, um das Atemschutzgerät (10) fest verbunden mit der Basis (20) in der Verriegelungsposition zu halten, wobei die Klinke (40) von einer Klinkenstütze (42) getragen und durch eine Feder (41) betätigt wird, die normalerweise auf einem vorderen Teil (45) der Klinke (40) aufliegt, um das fest verbundene Halten des Atemschutzgeräts (10) auf der Basis (20) in der Verriegelungsposition sicherzustellen.

2. Einheit nach einem der vorstehenden Ansprüche, **dadurch gekennzeichnet, dass** das Aufhängesystem (1, 11; 2, 21), erste Aufhängemittel (11) umfasst, getragen vom Atemschutzgerät (10), und zweite Aufhängemittel (2, 21), getragen von der Basis (20) und ausgelegt und entworfen, um mit den ersten Aufhängemitteln (1, 11) zusammenzuarbeiten, um eine Positionierung und eine Aufhängung des Atemschutzgeräts (10) an der Basis (20) zu ermöglichen.

3. Einheit nach einem der vorstehenden Ansprüche, **dadurch gekennzeichnet, dass** die Feder (41) mit einem Federkopf (43) ausgestattet ist, der normalerweise auf dem vorderen Teil (45) der Klinke (40) zum Aufliegen kommt, um sie in der Verriegelungsposition zu halten.

4. Einheit nach einem der vorstehenden Ansprüche, **dadurch gekennzeichnet, dass** der vordere Teil (45) der Klinke (40) eine abgerundete Form aufweist.

5. Einheit nach einem der vorstehenden Ansprüche, **dadurch gekennzeichnet, dass** der vordere Teil (45) der Klinke (40) eine Aufhängekralle (44) umfasst, die ausgelegt ist, um mit dem Anschlag (13) zusammenzuarbeiten, um ein fest verbundenes Halten des Atemschutzgeräts (10) auf der Basis (20) in der Verriegelungsposition sicherzustellen.

6. Einheit nach einem der vorstehenden Ansprüche, **dadurch gekennzeichnet, dass** der Anschlag (13) von der Stützplatte (1) getragen wird, die an die hintere Seite des Atemschutzegeräts (10) befestigt ist.

7. Einheit nach einem der vorstehenden Ansprüche, **dadurch gekennzeichnet, dass** der Anschlag (13) durch einen Teil der Wand der hinteren Stützplatte (1) des Atemschutzgeräts (10) gebildet ist.

8. Einheit nach einem der vorstehenden Ansprüche, **dadurch gekennzeichnet, dass** die hintere Platte (1) ein(e) zweite(s) Gehäuse oder Aussparung (12) umfasst, das/die abgemessen und konfiguriert ist, um die Klinke (40) aufzunehmen.

9. Einheit nach Anspruch 8, **dadurch gekennzeichnet, dass** ein Teil der Wand der hinteren Platte (1), der sich am unmittelbaren Umfang der zweiten Aussparung (12) befindet, den Anschlag (13) darstellt, auf dem die Verriegelungskralle (44) der Klinke (40) aufliegt, wenn sich das Atemschutzgerät (10) in der Verriegelungsposition befindet.

10. Einheit nach einem der vorstehenden Ansprüche, **dadurch gekennzeichnet, dass** sie Mittel zur Freisetzung (60, 6) umfasst, umfassend einen Hebel (6), der durch den Benutzer betätigt werden kann, der ausgelegt ist, um sich nach der Betätigung zu verschieben zwischen:
- mindestens einer ersten Verriegelungsposition, in der der Hebel (6) nicht auf die zweiten Verriegelungsmittel (40 - 45) wirkt, und sich das Atemschutzgerät (10) in der Verriegelungsposition befindet, und
- mindestens einer zweiten Freisetzungsposition, in der der Hebel (6) mit den zweiten Verriegelungsmitteln (40 - 45) interagiert, um das Atemschutzgerät (10) freizusetzen, um die Basis (20) entfernen zu können.

11. Einheit nach dem vorstehenden Anspruch, **dadurch gekennzeichnet, dass**, wenn der Hebel (6) vom Benutzer betätigt wird, der Hebel (6) wirkt, indem er eine Verriegelung der mechanischen und elektrischen Verbindungssysteme erzeugt.

12. Einheit nach einem der Ansprüche 10 oder 11, **dadurch gekennzeichnet, dass** der Hebel (6) durch Schwenken die Klinke (40) mit Hilfe einer geschweißten Muffe (61) betätigt.

13. Basis (20) nach Anspruch 1 einer Einheit nach einem der vorstehenden Ansprüche, **dadurch gekennzeichnet, dass** sie ein Fixierungssystem (31) umfasst, das seine direkte oder indirekte Fixierung an eine Wand umfasst, zweite Aufhängemittel (2, 21), zweite Verriegelungsmittel (40 - 45) und zweite elektrische Schützmittel (51).

## Claims

1. Respirator/baseplate unit comprising a respirator (10) and a baseplate (20) capable of and designed for receiving and supporting the respirator (10), said baseplate (20) comprising an attachment system (31) enabling the attachment thereof, direct or indirect, to a wall, said respirator/baseplate unit further comprising:
- a fastening system (1, 11; 2, 21) enabling a coupling positioning of the respirator (10) on the baseplate (20),
- a locking system (1, 12, 13; 40-45) enabling the respirator (10) to be locked in a locking position on the baseplate (20), subsequently to the coupling positioning, the locking position being separate from the coupling position, the locking system (1, 12, 13; 40-45) comprising first locking means (1, 12, 13) supported by the respirator (10), and second locking means (40-45) supported by the baseplate (20) and capable of and designed to cooperate with the first locking means (1, 12, 13) so as to enable a rigid attachment in locking position of the respirator (10) to the baseplate (20), and
- an electrical connection system (51-55) enabling to ensure an electrical connection between the respirator (1) and the baseplate (20), subsequently to the coupling positioning and simultaneously or quasi-simultaneously to the locking positioning, **characterised in that**:
- the first locking means (1, 12, 13) supported by the respirator (10) comprise a stopper (13), and
- the second locking means (40-45) supported by the baseplate (20) comprise a pivoting ratchet (40), said ratchet (40) cooperating with said stopper (13) to hold the respirator (10) rigid to the baseplate (20) in the locking position, the ratchet (40) being supported by a ratchet support (42) and actuated by a spring (41) which normally presses on a front part (45) of the ratchet (40) to ensure said rigid holding of the respirator (10) on the baseplate (20) in said locking position.

2. Unit according to one of the preceding claims, **characterised in that** the fastening system (1, 11; 2, 21) comprises first fastening means (11) supported by the respirator (10) and second fastening means (2, 21) supported by the baseplate (20) and capable of and designed to cooperate with said first fastening means (1, 11) so as to enable a positioning and a fastening of the respirator (10) to the baseplate (20).

3. Unit according to one of the preceding claims, **characterised in that** the spring (41) is equipped with a spring head (43) normally pressing on the front part (45) of the ratchet (40) to hold in the locking position.

4. Unit according to one of the preceding claims, **characterised in that** the front part (45) of the ratchet (40) is a rounded shape.

5. Unit according to one of the preceding claims, **characterised in that** the front part (45) of the ratchet (40) comprises a fastening claw (44) capable of cooperating with the stopper (13) to ensure the respirator (10) is rigidly held on the baseplate (20) in the locking position.

6. Unit according to one of the preceding claims, **characterised in that** the stopper (13) is supported by the support frame (1) attached to the rear face of the respirator (10).

7. Unit according to one of the preceding claims, **characterised in that** the stopper (13) is formed by a part of the rear support frame (1) wall of the respirator (10).

8. Unit according to one of the preceding claims, **characterised in that** the rear frame (1) comprises a second housing or recess (12) sized and configured to receive the ratchet (40).

9. Unit according to claim 8, **characterised in that** a part of the wall of said rear frame (1) located in the immediate periphery of the second recess (12) constitutes the stopper (13) whereon the fastening claw (44) presses on the ratchet (40), when the respirator (10) is in the locking position.

10. Unit according to one of the preceding claims, **characterised in that** it comprises release means (60, 6) comprising a lever (6) that can be actuated by the user, capable of being moved, after actuation, between:
- at least one first locking position, wherein the level (6) does not act on the second locking means (40-45) and the respirator (10) is in the locking position, and
- at least one second release position, wherein the level (6) interacts with the second locking means (40-45) to release the respirator (10) so as to be able to withdraw from the baseplate (20).

11. Unit according to the preceding claim, **characterised in that** when the lever (6) is actuated by the user, said lever (6) acts by causing the mechanical and electrical connection systems to unlock.

12. Unit according to one of claims 10 or 11, **characterised in that** the lever (6), by pivoting, actuates the ratchet (40) by the intermediary of a welded sleeve (61).

13. Unit (20) according to claim 1 of a unit according to one of the preceding claims, **characterised in that** it comprises an attachment system (31) enabling the attachment thereof, direct or indirect, to a wall, of the second fastening means (2, 21), the second locking means (40-45) and the second electric contactor means (51).
